# EUROPEAN PATENT APPLICATION

(11) **EP 4 749 639 A1**
(43) Date of publication of application: **27.05.2026**
(21) Application number: 25216160.9
(22) Date of filing: 17.11.2025
(51) Int. Cl.: G16H 20/40, A61B 6/51, G16H 30/40, G16H 50/50

(54) **SUPPORT APPARATUS, SUPPORT METHOD, AND SUPPORT PROGRAM**

(30) Priority: 26.11.2024 JP 2024205189
(71) Applicant: J. MORITA MANUFACTURING CORPORATION, Fushimi-ku, Kyoto-shi, Kyoto 6128533 (JP)
(72) Inventor: YOSHIKAWA, Hideki, Kyoto, 612-8533 (JP); KAJI, Ryosuke, Kyoto, 612-8533 (JP); ASANO, Teppei, Kyoto, 612-8533 (JP); NISHIMURA, Mikinori, Kyoto, 612-8533 (JP)
(74) Representative: Müller Hoffmann & Partner

(57) **Abstract**

A support apparatus is configured to support a procedure using a dental implant including a plurality of members. The support apparatus includes: an acquisition unit configured to acquire three-dimensional data representing a three-dimensional geometry of an interior of an oral cavity including teeth forming a dentition and a jawbone; a computing unit configured to determine a position for each of the plurality of members in the oral cavity based on the three-dimensional data acquired by the acquisition unit, and adjust the determined position of at least one of the plurality of members based on a positional relationship between the plurality of members; and an output unit configured to output support information representing a position for each of the plurality of members after the adjustment performed by the computing unit.

## Description

### CROSS REFERENCE TO RELATED APPLICATIONS

This nonprovisional application is based on Japanese Patent Application No. 2024-205189 filed on November 26, 2024 with the Japan Patent Office.

### BACKGROUND

### Field

The present disclosure relates to a support apparatus, a support method, and a support program for supporting a procedure using a dental implant including a plurality of members.

### Description of the Background Art

A conventionally known technique employs a three-dimensional scanner to scan a surface geometry of teeth, gingiva and the like in an oral cavity, a computed tomography (CT) scanner to CT scan a dentition, a jawbone and the like, or the like to obtain three-dimensional data representing a three-dimensional geometry in the oral cavity. For example, Japanese Patent Laying-Open No. 2020-096691 discloses using a three-dimensional scanner to obtain three-dimensional data representing a three-dimensional geometry of a tooth.

### SUMMARY

An operator such as a dentist acquires three-dimensional data of a tooth through the three-dimensional scanner disclosed in Japanese Patent Laying-Open No. 2020-096691 and uses the obtained three-dimensional data to produce an artificial tooth (hereinafter also referred to as a "dental implant") to compensate for a missing tooth. In a procedure (orthodontics) using such a dental implant, a position at which a dental implant is attached and the like are generally determined based on the operator's experiences. While a condition in an oral cavity in which a dental implant is attached varies for each patient, it is not preferable that a position at which the dental implant is attached significantly varies depending on the operator's experiences. There is a need for a technique allowing an operator to optimally perform a procedure using a dental implant regardless of the operator's experiences.

The present disclosure has been made to solve the above problem, and an object of the present disclosure is to provide a technique that can be used to optimally perform a procedure using a dental implant.

According to one example of the present disclosure, a support apparatus configured to support a procedure using a dental implant including a plurality of members is provided. The support apparatus comprises: an acquisition unit configured to acquire three-dimensional data representing a three-dimensional geometry of an interior of an oral cavity including teeth forming a dentition and a jawbone; a computing unit configured to determine a position for each of the plurality of members in the oral cavity based on the three-dimensional data acquired by the acquisition unit, and adjust the determined position of at least one member of the plurality of members based on a positional relationship between the plurality of members; and an output unit configured to output support information representing a position for each of the plurality of members after the adjustment performed by the computing unit.

According to one example of the present disclosure, a method for supporting by a computer a procedure using a dental implant including a plurality of members is provided. The method comprises, as steps performed by the computer: acquiring three-dimensional data representing a three-dimensional geometry of an interior of an oral cavity including teeth forming a dentition and a jawbone; determining a position for each of the plurality of members in the oral cavity based on the three-dimensional data acquired; adjusting, based on a positional relationship between the plurality of members, the position of at least one member of the plurality of members determined in the step of determining; and outputting support information representing a position for each of the plurality of members after the adjustment.

According to one example of the present disclosure, there is provided a support program configured to support by a computer a procedure using a dental implant including a plurality of members. The support program causes the computer to perform the steps of: acquiring three-dimensional data representing a three-dimensional geometry of an interior of an oral cavity including teeth forming a dentition and a jawbone; determining a position for each of the plurality of members in the oral cavity based on the three-dimensional data acquired; adjusting, based on a positional relationship between the plurality of members, the position of at least one member of the plurality of members determined in the step of determining; and outputting support information representing a position for each of the plurality of members after the adjustment.

These and other objects, features, aspects, and advantages of the present disclosure will become apparent from the following detailed description for the present disclosure, which is understood in conjunction with the accompanying drawings.

### BRIEF DESCRIPTION OF THE DRAWINGS

Fig. 1 shows an example of applying a support apparatus according to a first embodiment.
Fig. 2 is a block diagram for illustrating a hardware configuration of the support apparatus according to the first embodiment.
Fig. 3 is a diagram for illustrating a procedure using a dental implant.
Fig. 4 illustrates estimation of a position and a posture for attachment of a dental implant.
Fig. 5 is a diagram for illustrating an example of training an estimation model for estimating a partial optimal position for a top structure.
Fig. 6 is a diagram for illustrating an example of training an estimation model for estimating a partial optimal position for an abutment.
Fig. 7 is a diagram for illustrating an example of training an estimation model for estimating a partial optimal position for a fixture.
Fig. 8 is a diagram for illustrating an example of learning by an estimation model for estimating an overall optimal position.
Fig. 9 is a flowchart of support processing performed by the support apparatus.

### DESCRIPTION OF THE PREFERRED EMBODIMENTS

### <First Embodiment>

A first embodiment of the present disclosure will now be described in detail with reference to the drawings. In the figures, identical or equivalent components are identically denoted and will not be described repeatedly.

### [Exemplary Application]

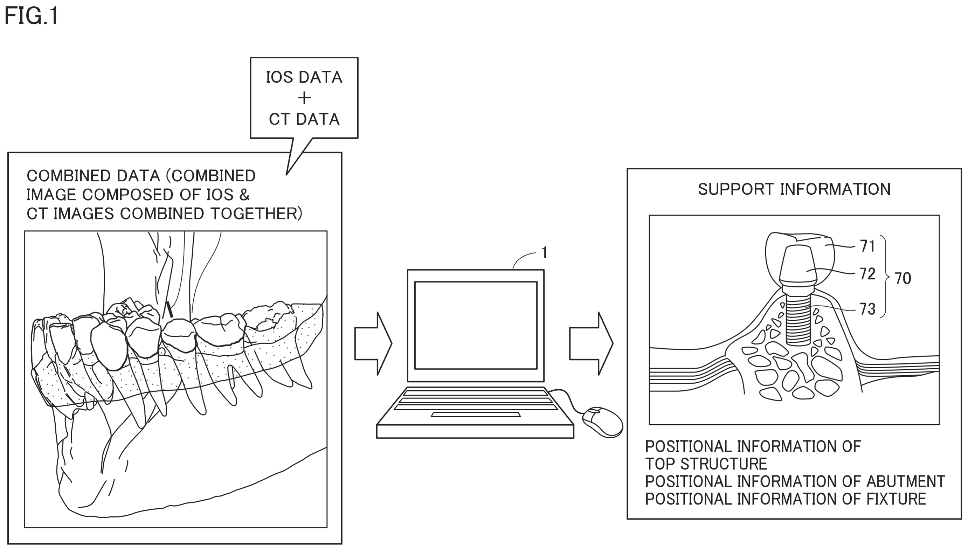

An exemplary application of support apparatus 1 according to the first embodiment will be described with reference to Fig. 1. Fig. 1 shows an example of applying support apparatus 1 according to the first embodiment.

As a treatment for a missing tooth or the like, a procedure using a dental implant 70 is generally performed. For example, dental implant 70 includes a top structure (an artificial tooth) 71, an abutment (a coupling portion) 72, and a fixture 73 (an artificial tooth root). A flow of treatment through a dental implant procedure is generally as follows: fixture 73 made of a base material adaptable to a human body is buried in a portion of a jawbone having a missing tooth, and top structure 71 is attached to an upper portion of fixture 73 via abutment 72. Fixture 73 and top structure 71 may be coupled together by abutment 72. Note that dental implant 70 may exclude abutment 72 and only include top structure 71 and fixture 73.

Where dental implant 70 is attached and what type of dental implant 70 is used should be appropriately selected while the patient's oral condition is considered. However, where dental implant 70 is attached and what type of dental implant 70 is selected may vary depending for example on an aspect that a surgical operator who performs a surgical procedure considers important, and the operator's skills and experiences.

Specifically, a position and a posture for fixture 73 relative to a jawbone, a length by which fixture 73 is buried in the jawbone, a position and a posture for abutment 72, a position and a posture for top structure 71, and the like may vary among operators. Furthermore, different operators may select different types of top structure 71, abutment 72, and fixture 73. For example, in general, an operator considers that it is important that a patient is relived from pain and dental implant 70 is tolerant, and accordingly, the operator determines a position and a posture for each member of dental implant 70 while considering a total balance for dental implant 70. In contrast, an operator who places importance on aesthetics for teeth for cosmetic surgery or the like may first determine a position and a posture for top structure 71 and then determine a position and a posture for fixture 73 with reference to the determined position and posture of top structure 71. Thus, a position, a posture, a type, and the like for each member of dental implant 70 may vary depending on various factors such as the operator's experiences, an aspect of and purpose of a dental implant procedure, and the like.

Accordingly, support apparatus 1 according to the first embodiment is configured to employ artificial intelligence (AI) technology to estimate (or derive) support information for supporting a procedure using dental implant 70.

Specifically, a user of support apparatus 1 uses a CT scanner (not shown) to CT scan a dentition and a jawbone in an oral cavity to acquire three-dimensional data representing a biological tissue including the jawbone in the oral cavity.

A "user" includes an operator (a medical practitioner or the like) or an assistant (a dental assistant, a dental technician, a nurse or the like) in various fields such as dentistry, oral surgery, orthopedics, plastic surgery, and cosmetic surgery. A "patient" includes a patient of dentistry, oral surgery, orthopedics, plastic surgery, and cosmetic surgery. The user uses a CT scanner (not shown) to scan a maxilla and mandible of a patient to acquire three-dimensional volumetric (voxel) data representing a hard tissue portion (a bone, a tooth, or the like) located around the maxilla and mandible of the patient, and including a soft tissue portion (skin, gingiva, or the like). The CT scanner is X-ray equipment that takes a CT of the patient's maxilla and mandible by rotating a transmitter and a receiver of X rays, which are one type of radioactive rays, around the patient's face. The user uses volumetric data of a target to be imaged, or a biological tissue, that is acquired through the CT scanner, to generate three-dimensional data representing the biological tissue. Note that the soft tissue portion is detected with X rays less clearly than the hard tissue portion, and data of the former is obtained less accurately than that of the latter. For this reason, when the soft tissue portion is displayed in an image, the soft tissue portion may have an insufficiently visible or invisible portion.

Furthermore, the user can use the volumetric data to generate a rendered image (a tomographic image or an appearance image) showing a three-dimensional geometry of the biological tissue. The "rendered image" is an image generated by processing or editing some data. Hereinafter, three-dimensional volumetric data acquired through a CT scanner will also be referred to as "CT data", and a rendered image generated based on the CT data will also be referred to as a "CT image". For example, a user can process or edit the volumetric data of the biological tissue acquired through the CT scanner to generate a rendered image showing the biological tissue in two dimensions as viewed at a predetermined point of view (a portion of biological tissue that can be represented by CT data), and furthermore, the user can change the predetermined point of view in multiple directions to generate a plurality of rendered images showing the biological tissue in two dimensions as viewed in the multiple directions (the portion of biological tissue that can be represented by CT data).

Furthermore, the user may use a three-dimensional scanner (an optical scanner) (not shown) to scan the patient's oral cavity to acquire optical scanner data including positional information of each point of point cloud data (a plurality of points) representing a surface of a biological tissue including teeth and gingiva in the oral cavity. The optical scanner data includes, as the positional information, coordinates (X, Y, Z) of each point representing the surface of the biological tissue in a lateral direction (a direction along the X-axis), a longitudinal direction (a direction along the Y-axis), and a height-wise direction (a direction along the Z-axis) that are predetermined. Furthermore, the optical scanner data may include color information representing an actual color of a portion corresponding to each point of the point cloud data (the plurality of points) representing the surface of the biological tissue including the teeth and the gingiva in the oral cavity (a surface portion of the biological tissue).

The three-dimensional scanner is a so-called intraoral scanner (IOS) capable of optically imaging an interior of an oral cavity of a patient in a confocal method, through triangulation, or the like, and is capable of acquiring positional information of each point of point cloud data that defines a surface of a biological tissue (e.g., a tooth and gingiva in the oral cavity) set in some coordinate space to be scanned. The user can use the optical scanner data acquired through the three-dimensional scanner to generate a rendered image (an appearance image) showing a three-dimensional geometry of the biological tissue. Hereinafter, optical scanner data acquired through a three-dimensional scanner and including positional information of each point of point cloud data representing a surface of a biological tissue will also be referred to as "IOS data", and a rendered image generated based on the IOS data will also be referred to as "IOS image". For example, the user can process or edit optical scanner data acquired through a three-dimensional scanner and representing a biological tissue to generate a rendered image showing the biological tissue in two dimensions as viewed at a predetermined point of view (a portion of biological tissue that can be represented by IOS data). Furthermore, the user can change the predetermined point of view in multiple directions to generate a plurality of rendered images showing the biological tissue in two dimensions as viewed in the multiple directions (the portion of biological tissue that can be represented by the IOS data). Alternatively, the three-dimensional scanner may be optical scanner data acquired through a desktop scanner.

While an IOS image can show in a very detailed manner a surface geometry of a biological tissue to be scanned, the IOS image cannot show an internal construction that does not appear on the surface of the biological tissue (such as alveolar bone and a root apex). In contrast, while a CT image can show relatively in detail a hard tissue portion (such as bone, tooth, etc.) of a target to be imaged, the CT image cannot show a soft tissue portion (such skin, gingiva, etc.) in more detail than the hard tissue portion.

The user may combine IOS data and CT data acquired for the same patient. Combining the IOS data and the CT data together generates combined data. The IOS data and the CT data have different data formats. Accordingly, the IOS and CT data are commonized. For example, the user may convert the data format of the IOS data into the data format of the CT data and use both of the converted data to subject a three-dimensional geometry of a surface of a biological tissue to pattern-matching to generate combined data composed of the IOS data and the CT data combined together. Alternatively, the user may generate combined data composed of the IOS data and the CT data combined together with reference to a position of a tooth crown in the IOS data and that of the tooth crown in the CT data. The user may convert the data format of the CT data into the data format of the IOS data and use both of the converted data to subject a three-dimensional geometry of a surface of some biological tissue to pattern-matching and use a matched position as a reference to recalculate a three-dimensional position for another biological tissue to generate combined data. The pattern-matching is done by subjecting the IOS data and the CT data to three-dimensional geometric pattern-matching for a crown of a tooth having a relatively large degree of matching. The IOS data and the CT data are acquired in different manners, and while they provide a high matching level, they may not match 100%, and accordingly, they may be regarded to be matched when they attain a predetermined level or higher (e.g., 95% or higher). Alternatively, the user may convert the data formats of the CT and IOS data into a common data format and use both of the converted data to subject a three-dimensional geometry of a surface of a biological tissue to pattern-matching to generate combined data. The user can process or edit the combined data to generate a rendered image showing the biological tissue in two dimensions as viewed at a predetermined point of view (a portion of biological tissue that can be represented by both the IOS data and the CT data) (e.g., the combined image shown in Fig. 1). Alternatively, rather than commonizing the data, the user may cause data to be generated in some three-dimensional space at corresponding coordinate positions of the IOS and CT data to generate combined data composed of both data combined together. While such a method of generating data is also referred to as overlay, fusion, or superposition, it is defined herein as combination in a broad sense. As shown in Fig. 1, the combined image can three-dimensionally show a surface geometry of a biological tissue represented by the IOS data and a tomographic structure or appearance of a hard tissue portion (bone, tooth, etc.) represented by the CT data. In generating the combined data, the user may adjust the IOS data and the CT data in brightness, contrast, transmittance, and the like, as necessary. Furthermore, the user may previously segment each of a plurality of teeth, a jawbone, and an alveolar bone in each of the IOS data and the CT data, and then generate combined data.

Note that support apparatus 1 may acquire CT data from a CT scanner and also acquire IOS data from a three-dimensional scanner, and follow an input of the user to generate combined data based on the acquired IOS and CT data. Alternatively, support apparatus 1 may not acquire IOS data and CT data and instead acquire from another apparatus combined data generated by the user using the other apparatus.

The combined image that can be generated based on the combined data allows the CT data to represent a three-dimensional geometry of a hard tissue portion such as an alveolar bone and a root apex and allows the IOS data to represent a three-dimensional geometry of a soft tissue portion such as gingiva that the CT data cannot represent. The combined image can thus also show in detail the soft tissue portion such as gingiva that cannot be represented by the CT data alone together with the hard tissue portion such as the alveolar bone and the root apex as the IOS data compensates for the soft tissue portion.

As will be described hereinafter in detail, support apparatus 1 estimates in combined data representing a biological tissue at least including a jawbone at least one of: a position at which each member of dental implant 70 is attached as appropriate; and a type of each member of dental implant 70. Support apparatus 1 uses estimation models 30A to 30D, which will be described hereinafter, to derive support information including information for at least one of the position at which dental implant 70 is attached and the type of the dental implant. Hereinafter will be described an example of estimating an appropriate position for attaching dental implant 70.

The support information as one example may be combined data showing a user and a patient an image obtained after a procedure using dental implant 70 ends, with three-dimensional data that represents dental implant 70 superimposed on the combined data at a location where a tooth is lost. Fig. 1 shows, as the support information, three-dimensional data superimposed on the combined data and representing dental implant 70. Support apparatus 1 may provide the user with numerical values representing a position and a posture for each member constituting dental implant 70 (i.e., top structure 71, abutment 72, and fixture 73) as the support information. For example, the support information includes positional information (X, Y, Z) of each member in an oral cavity or information representing an angle of each member in the oral cavity. That is, support apparatus 1 may provide the user with an image showing at least one of a position and a posture for each member as the support information or may provide the user with a numerical value representing at least one of a position and a posture for each member constituting dental implant 70 as the support information. This can help the user to determine where in the oral cavity of an actual patient each member of dental implant 70 can be attached appropriately.

### [Hardware Configuration of Support Apparatus]

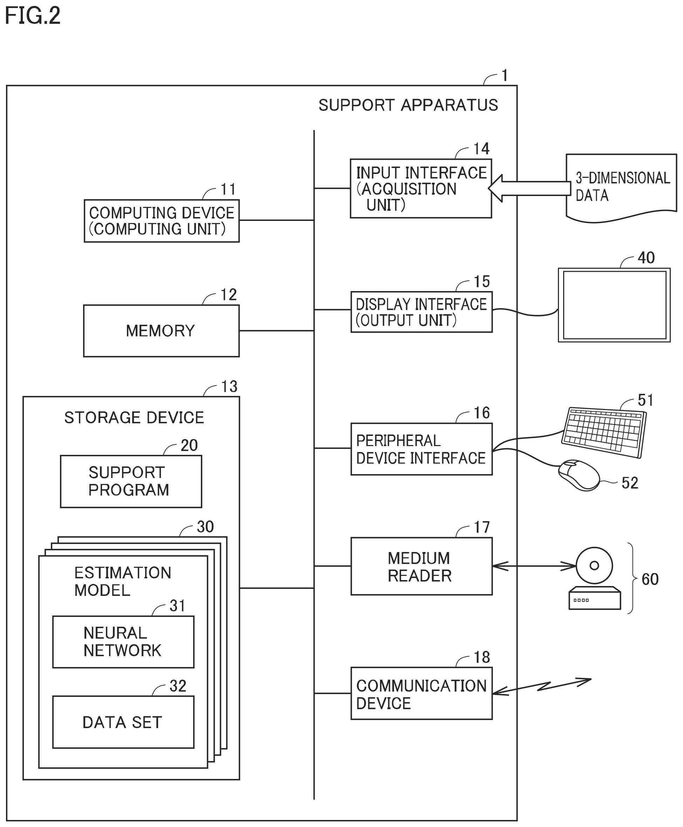

A hardware configuration of support apparatus 1 according to the first embodiment will now be described with reference to Fig. 2. Fig. 2 is a block diagram for illustrating the hardware configuration of support apparatus 1 according to the first embodiment. Support apparatus 1 may be implemented for example by a general-purpose computer, or a computer dedicated to a system for estimating the support information.

As shown in Fig. 2, support apparatus 1 comprises as its main hardware components a computing device 11, a memory 12, a storage device 13, an input interface 14, a display interface 15, a peripheral device interface 16, a medium reader 17, and a communication device 18.

Computing device 11 is a computing entity (a computer) that executes a variety of types of programs to perform a variety of types of processing, and is an example of a "computing unit". Computing device 11 is configured by a processor such as a central processing unit (CPU), a micro-processing unit (MPU), a neural network processing Unit (NPU), a tensor processing unit (TPU), or a graphics processing unit (GPU). Note that while the processor that is an example of computing device 11 has a function of performing a variety of types of processing by executing a program, these functions may partially or entirely be implemented using dedicated hardware circuitry such as an application specific integrated circuit (ASIC) or a field-programmable gate array (FPGA). The "processor" is not limited to a processor in a narrow sense, such as a CPU, an MPU, an NPU, a TPU, or a GPU, that performs processing in a stored program architecture, and the processor may include hardwired circuitry such as an ASIC or an FPGA. For this reason, the "processor" as an example of computing device 11 can also be read as processing circuitry which performs processing predefined by computer-readable code and/or hardwired circuitry. Note that computing device 11 may be composed of a single chip or a plurality of chips. Furthermore, the processor and associated processing circuitry may be composed of a plurality of computers interconnected in a wired or wireless manner via a local area network or a wireless network or the like. The processor and associated processing circuitry may be implemented by a cloud computer that remotely performs computation based on input data and outputs a result of computation to another remotely located device.

Memory 12 includes a volatile storage area (for example, a working area) that temporarily stores program code, a work memory, etc. when computing device 11 executes a variety of types of programs. Examples of memory 12 include volatile memory such as dynamic random access memory (DRAM) and static random access memory (SRAM) or non-volatile memory such as read only memory (ROM) and flash memory.

Storage device 13 stores a variety of types of programs that computing device 11 executes, a variety of types of data, and the like. Storage device 13 may be implemented by one or more non-transitory computer readable media or one or more computer readable storage media. Examples of storage device 13 include a hard disk drive (HDD) and a solid state drive (SSD).

Storage device 13 stores a support program 20 and an estimation model 30. Support program 20 describes contents of support processing for computing device 11 to use estimation model 30 to estimate support information based on image data (for example, combined data) representing a three-dimensional geometry of a biological tissue.

Estimation model 30 includes a neural network 31 and a data set 32 used by neural network 31. Estimation model 30 is trained by machine learning using training data including image data (e.g., combined data) representing a three-dimensional geometry of a biological tissue in an oral cavity and ground truth data associated with that image data (or combined data obtained after dental implant 70 is actually attached) to estimate support information based on combined data representing a state with at least one missing tooth.

Any algorithm may be applied to neural network 31 insofar as the algorithm is applicable to neural network 31 of the first embodiment, such as an autoencoder, a convolutional neural network (CNN), a recurrent neural network (RNN), a transformer, a state space model, or a generative adversarial network (GAN). Estimation model 30 is not limited to neural network 31 and may include another known algorithm such as Bayesian estimation or support vector machine (SVM).

Data set 32 includes a weighting coefficient used for computation by neural network 31, and a determination threshold used for determination made when computation is performed.

Input interface 14 is an example of an "acquisition unit". Input interface 14 for example acquires combined data representing an interior of an oral cavity. The combined data received through input interface 14 is stored in memory 12 or by storage device 13, and used when computing device 11 estimates support information. Note that input interface 14 may acquire CT data and IOS data before they are combined together. For example, input interface 14 may be communicably connected to a three-dimensional scanner (not shown) to acquire IOS data from the three-dimensional scanner. Input interface 14 may be communicably connected to a CT scanner (not shown) to acquire CT data from the CT scanner. In that case, computing device 11 combines the IOS and CT data that are received through input interface 14 together to generate the above-described combined data, and estimates support information based on the generated combined data. Note that the data used for estimating the support information is not limited to the combined data, and may for example be CT data obtained before it is combined. That is, support apparatus 1 may not use IOS data and instead use CT data alone to estimate support information.

Display interface 15 is an example of an "output unit" and it is an interface for connecting a display 40. Display interface 15 implements inputting and outputting data between support apparatus 1 and display 40. Display interface 15 outputs image data to display 40 to represent support information estimated through estimation model 30 and causes display 40 to display the support information.

Peripheral device interface 16 is an interface for connecting peripheral devices such as a keyboard 51 and a mouse 52. Peripheral device interface 16 implements inputting and outputting data between support apparatus 1 and peripheral devices.

Medium reader 17 reads a variety of types of data stored in a storage medium 60 and writes a variety of types of data to storage medium 60. For example, medium reader 17 may acquire support program 20 from storage medium 60 or may write to storage medium 60 support information estimated by computing device 11. Storage medium 60 may be implemented by one or more non-transitory computer readable media or one or more computer readable storage media. When computing device 11 acquires image data (e.g., combined data) from storage medium 60 via medium reader 17, medium reader 17 can be an example of the "acquisition unit".

Communication device 18 transmits and receives data to and from an external device (not shown) through wired or wireless communication. For example, communication device 18 may transmit support information estimated by computing device 11 to the external device. When computing device 11 acquires image data (e.g., combined data) from the external device via communication device 18, communication device 18 can be an example of the "acquisition unit". When support information estimated through estimation model 30 is output to an external device different from display 40, communication device 18 can be an example of the "output unit".

### [Procedure Using Dental Implant]

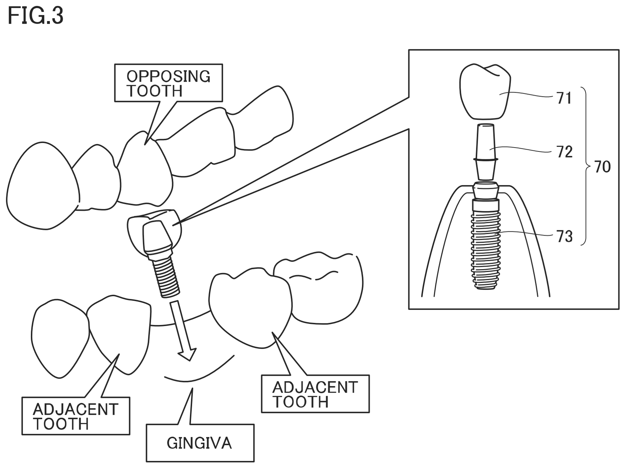

Fig. 3 is a diagram for illustrating a procedure using dental implant 70. As shown in Fig. 3, dental implant 70 includes top structure 71, abutment 72, and fixture 73, each as an independent component. Dental implant 70 is attached for example to a defect portion with a tooth lost and gingiva exposed. Initially, fixture 73 is buried in and fixed to a jawbone covered with gingiva, and top structure 71 is attached to fixture 73 via abutment 72. Dental implant 70 is attached in an oral cavity in such a manner as to keep an optimal position and posture while the position and posture of a tooth adjacent to a surgical site, and the position and posture of a tooth opposing the surgical site and the state of the jawbone are considered.

### [Estimating Position and Posture for Attachment of Dental Implant]

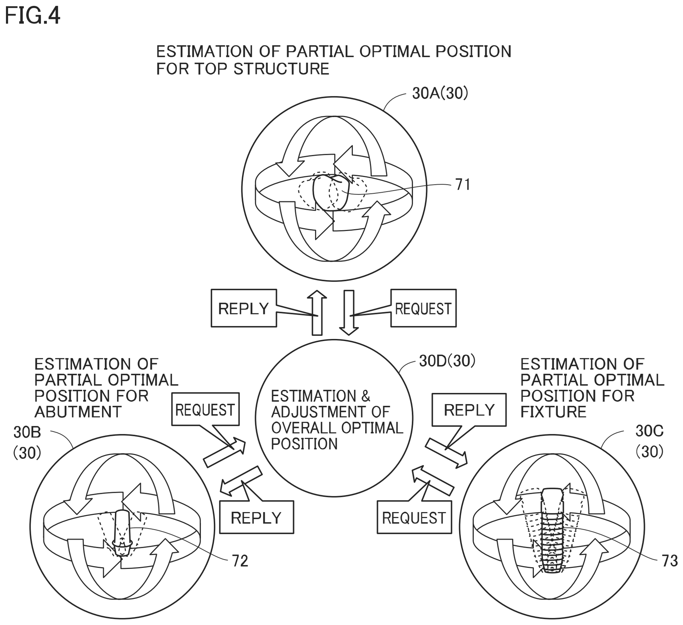

Fig. 4 is a diagram for illustrating how a position and a posture is estimated for attachment of dental implant 70. Computing device 11 of support apparatus 1 determines an optimal position for each of the plurality of members of dental implant 70 based on three-dimensional data representing a state with a missing tooth. As illustrated in Fig. 4, computing device 11 uses estimation models 30A, 30B and 30C to estimate optimal positions for top structure 71, abutment 72, and fixture 73, respectively. Note that estimation models 30A, 30B and 30C are an example of a "first estimation model" in the present disclosure. That is, computing device 11 uses estimation models 30A to 30C to estimate an optimal position for each member independently. It should be noted that, in addition to the process for estimating an optimal position for each member, estimation models 30A to 30C can also perform a process for adjusting the optimal position estimated for the member. Hereinafter, an optimal position estimated through estimation model 30A, 30B, 30C for each member will be referred to as a "partial optimal position". Estimation models 30A, 30B and 30C are included in estimation model 30 illustrated in Fig. 2.

A partial optimal position is a position estimated for each member (top structure 71, abutment 72, and fixture 73) based on three-dimensional data representing a state with a missing tooth and is a position without consideration for a relationship with the other members. That is, a partial optimal position for top structure 71 is estimated by estimation model 30A without considering a position for abutment 72 and a position for fixture 73. Similarly, a partial optimal position for abutment 72 is estimated by estimation model 30B without considering a position for top structure 71 and a position for fixture 73. Furthermore, a partial optimal position for fixture 73 is estimated by estimation model 30C without considering a position for top structure 71 and a position for abutment 72. The partial optimal position for each member is estimated while a predetermined aspect for the member is considered. The predetermined aspect includes at least one of: pain of a patient to be treated; each member's tolerance; occlusion of dentition of the patient; and the patient's oral aesthetics. For example, the partial optimal position for top structure 71 is estimated such that top structure 71 is optimally positioned in view of aesthetics and occlusion. Furthermore, the partial optimal positions for abutment 72 and fixture 73 are estimated such that abutment 72 and fixture 73 are optimally positioned in view of the patient's pain and each member's tolerance. That is, top structure 71 accommodates aesthetics and occlusion, and abutment 72 and fixture 73 accommodate pain and tolerance. Note that each member may not accommodate a predetermined aspect as described above, and the member may accommodate a different aspect or may accommodate a different combination of aspects. For example, top structure 71 may accommodate tolerance and fixture 73 may accommodate occlusion.

The partial optimal position may for example be a position with reference to a jawbone and may specifically be three-dimensional data or a numerical value including a distance between the jawbone and each member and each member's posture (or angle) relative to the jawbone. Estimation models 30A to 30C independently estimate partial optimal positions for the respective members based on predetermined aspects that the members accommodate. Therefore, when the members are disposed at partial optimal positions that are a result output from estimation models 30A to 30C, the members may not be assemblable as one dental implant 70. For example, the members may have their respective partial optimal positions with an excessively large distance therebetween or overlapping in a three-dimensional coordinate system, or may not have their respective axes on a single axis.

Accordingly, computing device 11 according to the first embodiment uses an estimation model 30D to adjust the partial optimal positions that are estimated independently of one another by estimation models 30A to 30C to be positions allowing the members to be assembled as one dental implant 70. Estimation model 30D is an example of a "second estimation model" in the present disclosure. Specifically, computing device 11 uses estimation model 30D to handle as a set the three members of top structure 71, abutment 72, and fixture 73 constituting dental implant 70 and estimate an optimal position while considering the entire dental implant 70. Hereinafter, an optimal position estimated through estimation model 30D for the entire dental implant 70 will also be referred to as an "overall optimal position". The overall optimal position includes positional information of each of top structure 71, abutment 72 and fixture 73, and is a position allowing the members to be assembled together to be one dental implant 70. The overall optimal position estimated through estimation model 30D is a position in which the positions of top structure 71, abutment 72, and fixture 73 are mutually considered.

Estimation model 30D is a model that learns using, as ground truth data, three-dimensional data obtained after a specific operator actually applies a dental implant procedure to a specific patient and representing the interior of the oral cavity of the specific patient. Therefore, estimation model 30D is configured to output an output result reflecting a result of an actual treatment performed by the operator who conducted the procedure represented by the three-dimensional data serving as the ground truth data. Hereinafter, estimation models 30A, 30B, 30C and 30D may collectively be referred to as "estimation model 30", and estimation models 30A, 30B, 30C and 30D may each be referred to as "estimation model 30".

After computing device 11 estimates an overall optimal position through estimation model 30D, the computing device segments the members, compares the positions of the members in the overall optimal position with the partial optimal positions of the members estimated independently of one another through estimation models 30A to 30C, and adjusts the partial optimal positions of the members estimated through estimation models 30A to 30C. Specifically, estimation models 30A to 30C each inquire whether the partial optimal position that the estimation model estimates is an optimal position when the estimated partial optimal position is compared with the overall optimal position estimated through estimation model 30D. In other words, estimation models 30A to 30C each request estimation model 30D to reply for whether the partial optimal position that estimation model 30A, 30B, 30C estimates is an optimal position when the total balance of the entire dental implant 70 is considered. In response, estimation model 30D compares the overall optimal position with the partial optimal position acquired from each of estimation models 30A to 30C, and generates a reply for whether the partial optimal position is an optimal position when the total balance of the entire dental implant 70 is considered. The reply includes a result of whether the partial optimal positions received from estimation models 30A to 30C are optimal, and information for adjusting a partial optimal position when the partial optimal position is not optimal. The information for adjusting a partial optimal position includes post-adjustment positional information of the partial optimal position allowing the partial optimal position to be an optimal position when the total balance of the entire dental implant 70 is considered. By repeating such a request and replay between each of estimation models 30A to 30C and estimation model 30D, computing device 11 adjusts each partial optimal position to a position allowing the members to be assembled as one dental implant 70. Hereinafter, a method for training estimation model 30 for each of estimation models 30A to 30D will be described in detail.

### [Training Estimation Models]

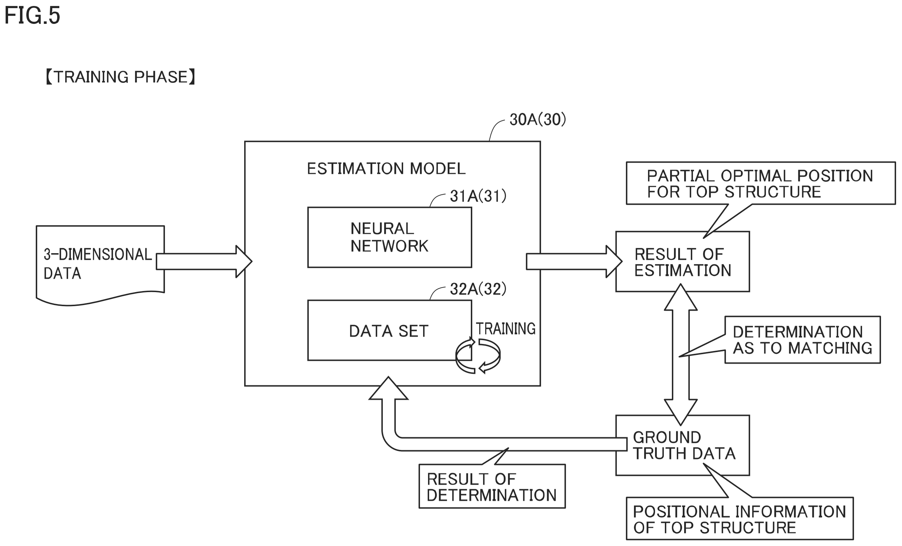

How estimation models 30A to 30D are trained through machine learning will now be described with reference to Figs. 5 to 8.

Fig. 5 is a diagram for illustrating an example of training estimation model 30A that estimates a partial optimal position for top structure 71. As illustrated in Fig. 5, in the first embodiment, three-dimensional data representing a three-dimensional geometry of an interior of an oral cavity including teeth forming a dentition and a jawbone is included in training data. For example, when estimation model 30A is trained, machine learning is performed using the three-dimensional data and ground truth data including a position and a posture for top structure 71. The ground truth data including a position and a posture for top structure 71 is, for example, three-dimensional data representing a state with a missing tooth, together with an representation of a position of top structure 71 actually applied by an operator who considers aesthetics important. The ground truth data may be data representing the position of top structure 71, and may not be three-dimensional data and instead be numerical data representing positional information and a posture. The three-dimensional data representing the position of top structure 71 with aesthetics considered important for example includes three-dimensional data representing: a position allowing formation of a U-shaped dental arch that is considered to be beautiful; a position allowing an adjacent tooth to match in height; a position allowing an optimal interdental space, rather than an excessively large interdental space; a position allowing a tooth root to be unexposed from gingiva and a tooth crown to be alone exposed; and the like.

An aspect reflected in an output result may vary when ground truth data is obtained from different sources. Generally, surgeons in the field of cosmetic surgery tend to consider aesthetics more important than surgeons in other fields do. For example, when estimation model 30A is trained on ground truth data which is three-dimensional data including a position and posture of top structure 71 actually applied by an operator who considers an aesthetic appearance important, estimation model 30A can estimate such a position and posture for top structure 71 that places importance on the aesthetic appearance. Furthermore, when estimation model 30A is trained on ground truth data which is three-dimensional data including a position and posture of top structure 71 actually applied by an operator who considers occlusion important, estimation model 30A can estimate such a position and posture for top structure 71 that places importance on occlusion.

When estimation model 30A acquires three-dimensional data representing a state with a missing tooth, the estimation model estimates a partial optimal position for top structure 71 by a neural network 31A based on the input three-dimensional data. In doing so, estimation model 30A estimates a position and posture optimal for top structure 71 without considering those for the other members constituting dental implant 70 (i.e., abutment 72 and fixture 73). The position and posture optimal for top structure 71 is, for example, a position and a posture that take into account the position and posture of a tooth adjacent to the surgical site, and the position and posture of an opposing tooth facing the surgical site. Neural network 31A is included in neural network 31 shown in Fig. 2. Estimation model 30A determines whether the estimated partial optimal position of top structure 71 matches the positional information of top structure 71 that is the ground truth data associated with the input three-dimensional data. When they match, estimation model 30A does not update a data set 32A, whereas when they do not match, the estimation model updates data set 32A to optimize data set 32A. Data set 32A is included in data set 32 shown in Fig. 2. Optimizing data set 32A includes setting parameters included in data set 32A, such as a threshold value and a weighting coefficient for aesthetics, so that top structure 71 is positioned with aesthetics considered important. Neural network 31A optimizes data set 32A to set priority for a predetermined aspect.

Thus, estimation model 30A uses training data including input data that is three-dimensional data representing a state with a missing tooth before a dental implant procedure is performed for the missing tooth and ground truth data that is positional information of top structure 71 to optimize data set 32A so that the estimation model is trained to be able to estimate a partial optimal position for top structure 71 with high accuracy.

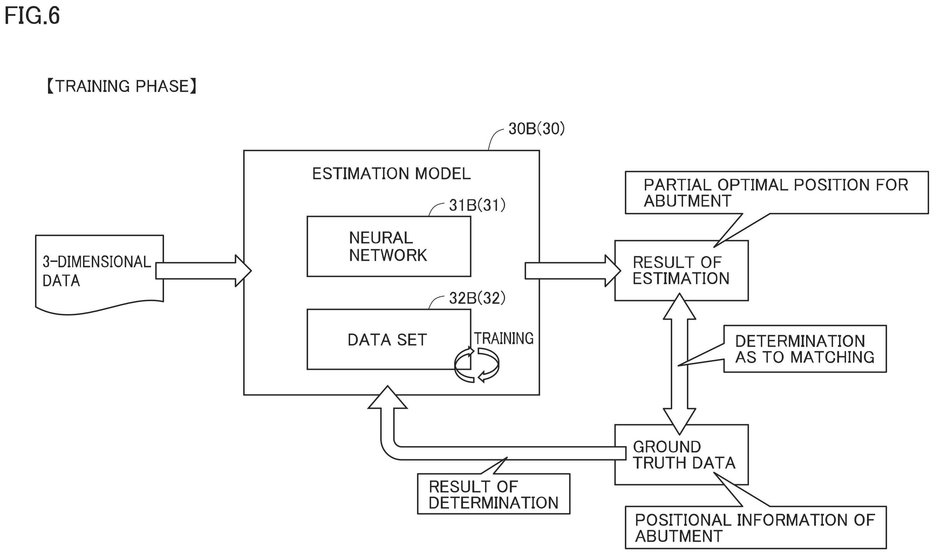

Fig. 6 is a diagram for illustrating an example of training estimation model 30B that estimates a partial optimal position for abutment 72. As illustrated in Fig. 6, training estimation model 30B also involves machine learning using training data including three-dimensional data representing a three-dimensional geometry of an interior of an oral cavity including teeth forming a dentition and a jawbone and ground truth data that is positional information of abutment 72 actually applied through an actually performed dental implant procedure.

When estimation model 30B is trained on ground truth data which is three-dimensional data including a position and posture of abutment 72 actually applied by an operator who places importance on aesthetics and relieving a patient from pain, estimation model 30B can estimate such a position and posture for abutment 72 that places importance on aesthetics and relieving a patient from pain. When estimation model 30B is trained on ground truth data which is three-dimensional data including a position and posture of abutment 72 actually applied by an operator who places importance on aesthetics with top structure 71 attached, and enhancing abutment 72 in tolerance, estimation model 30B can estimate such a position and posture for abutment 72 that places importance on aesthetics with top structure 71 attached, and enhancing abutment 72 in tolerance. As shown in Fig. 3, when abutment 72 is buried, the abutment is partially located in top structure 71 and accordingly, aesthetics with top structure 71 attached will be considered, and the abutment is also partially located in an alveolar bone or a jawbone and accordingly, tolerance and pain based on the alveolar bone's thickness and the jawbone's bone mass and bone density will be considered. When aesthetics is compared with tolerance and pain, tolerance and pain tend to be considered more important than aesthetics.

When estimation model 30B acquires the three-dimensional data representing the state with the missing tooth, the estimation model estimates a partial optimal position for abutment 72 by a neural network 31B based on the acquired three-dimensional data. In doing so, estimation model 30B estimates a position and posture optimal for abutment 72 without considering those for the other members constituting dental implant 70 (i.e., top structure 71 and fixture 73). The position and posture optimal for abutment 72 is, for example, a position and a posture that take into account tolerance that abutment 72 has after abutment 72 is attached, and pain that the patient suffers after abutment 72 is attached. Neural network 31B is also included in neural network 31 shown in Fig. 2.

Thus, estimation model 30B also optimizes a data set 32B by determining whether the estimated partial optimal position of abutment 72 matches the positional information of abutment 72, or the ground truth data. Thus, estimation model 30B is trained to be able to estimate a partial optimal position for abutment 72 with high accuracy. Optimizing data set 32B includes setting parameters included in data set 32B, such as a threshold value and a weighting coefficient for aesthetics, and tolerance and pain, so as to provide a position with aesthetics, and tolerance and pain considered important. Neural network 31B optimizes data set 32B to set priority for a predetermined aspect.

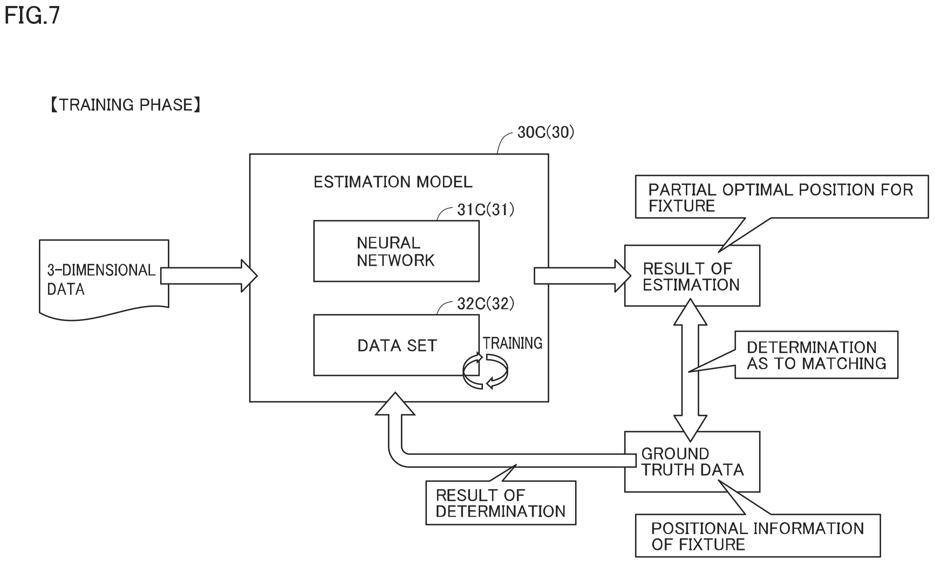

Fig. 7 is a diagram for illustrating an example of training estimation model 30C that estimates a partial optimal position for fixture 73. As illustrated in Fig. 7, training estimation model 30C also involves machine learning using training data including three-dimensional data representing a three-dimensional geometry of an interior of an oral cavity including teeth forming a dentition and a jawbone and ground truth data that is positional information of fixture 73 actually applied through an actually performed dental implant procedure.

When estimation model 30C is trained on ground truth data which is three-dimensional data including a position and posture of fixture 73 actually applied by an operator who places importance on relieving a patient from pain, and tolerance, estimation model 30C can estimate such a position and posture for fixture 73 that places importance on relieving a patient from pain, and tolerance. When estimation model 30C is trained on ground truth data which is three-dimensional data including a position and posture of fixture 73 actually applied by an operator who places importance on relieving a patient from pain and enhancing fixture 73 in tolerance, estimation model 30C can estimate such a position and posture for fixture 73 that places importance on relieving a patient from pain and enhancing fixture 73 in tolerance. A patient's pain may be affected by: excessive contact between top structure 71 and an adjacent tooth; fixture 73 buried excessively deep; thin alveolar bone; contact with a mandibular canal; small bone mass and low bone density nearby; and the like. The tolerance is affected, for example, by small bone mass and low bone density nearby.

When estimation model 30C acquires the three-dimensional data representing the state with the missing tooth, the estimation model estimates a partial optimal position for fixture 73 by a neural network 31C based on the acquired three-dimensional data. In doing so, estimation model 30C estimates a position and posture optimal for fixture 73 without considering those for the other members constituting dental implant 70 (i.e., top structure 71 and abutment 72). The position and posture optimal for fixture 73 is, for example, a position and a posture that take into account tolerance that fixture 73 has after fixture 73 is attached, pain that a patient suffers after fixture 73 is attached, and the like. Neural network 31C is also included in neural network 31 illustrated in Fig. 2.

Thus, estimation model 30C also optimizes data set 32C by determining whether the estimated partial optimal position of fixture 73 matches the positional information of fixture 73, or the ground truth data. Thus, estimation model 30C is trained to be able to estimate a partial optimal position for fixture 73 with high accuracy. Optimizing data set 32C includes setting parameters included in data set 32C, such as a threshold value and a weighting coefficient for tolerance and pain, so as to provide a position with tolerance and pain considered important. Neural network 31C optimizes data set 32C to set priority for a predetermined aspect.

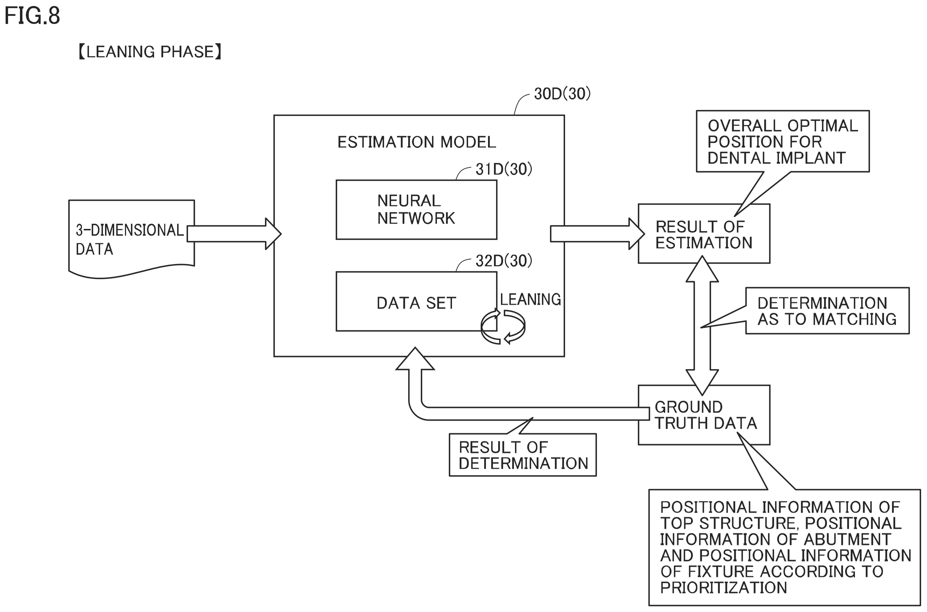

Subsequently, estimation model 30D configured to estimate an overall optimal position will be described. Fig. 8 is a diagram for illustrating an example of machine learning in a learning phase of estimation model 30D that estimates an overall optimal position. As illustrated in Fig. 8, in the first embodiment, training data includes three-dimensional data representing a state with at least one missing tooth, and ground truth data that is positional information of dental implant 70 (positional information of top structure 71, positional information of abutment 72, and positional information of fixture 73) obtained after a dental implant procedure is actually performed and the members are assembled together.

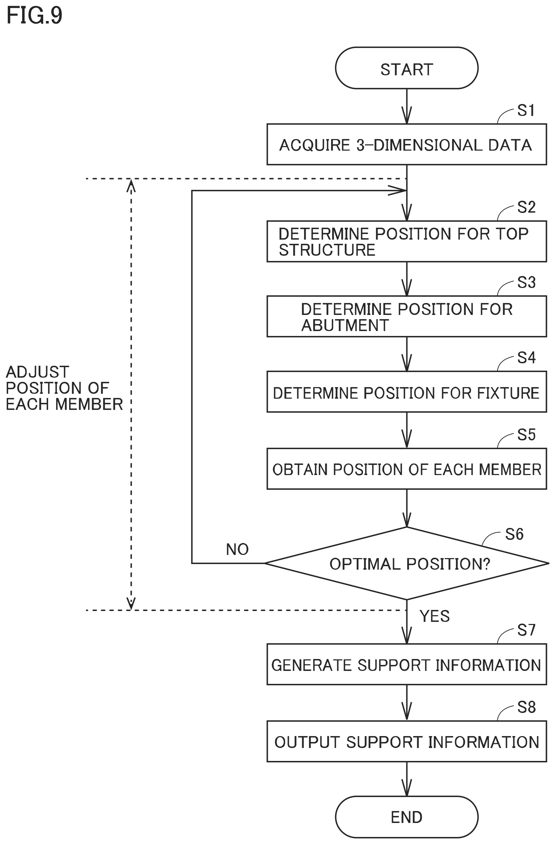

When estimation model 30D receives the three-dimensional data representing the state with the missing tooth, the estimation model estimates an overall optimal position for dental implant 70 by a neural network 31D based on the received three-dimensional data. Estimation model 30D also optimizes data set 32D by determining whether the estimated overall optimal position of dental implant 70 matches the ground truth data, that is, the positional information of top structure 71, the positional information of abutment 72, and the positional information of fixture 73. Thus, estimation model 30D is trained to be able to estimate an overall optimal position for dental implant 70 with high accuracy. Hereinafter, an example of adjusting a partial optimal position will be described with reference to a flowchart shown in Fig. 9.

### [Support Processing]

Fig. 9 is a flowchart of support processing performed by the support apparatus. The flowchart shown in Fig. 9 is implemented by computing device 11 executing support program 20.

Computing device 11 acquires three-dimensional data representing a three-dimensional geometry of an interior of an oral cavity including teeth forming a dentition and a jawbone (step S1). For example, the three-dimensional data represents a state of an interior of an oral cavity of a patient with a missing tooth subject to a dental implant procedure. Computing device 11 inputs the three-dimensional data acquired in step S1 to estimation model 30A to determine a partial optimal position for top structure 71 (step S2). Computing device 11 inputs the three-dimensional data acquired in step S1 to estimation model 30B to determine a partial optimal position for abutment 72 (step S3). Computing device 11 inputs the three-dimensional data acquired in step S1 to estimation model 30C to determine a partial optimal position for fixture 73 (step S4). Note that computing device 11 may perform steps S2 to S4 simultaneously or in an order different from that represented in the Fig. 9 example. Computing device 11 acquires from estimation models 30A to 30C a positional relationship of the members determined through steps S2, S3 and S4 (step S5). That is, in step S5, computing device 11 acquires information representing positions and postures for the partial optimal positions of the members acquired in steps S2 to S4.

Computing device 11 determines whether the partial optimal positions of the members determined in steps S2 to S4 are an optimal position when the members are viewed as a whole (step S6). Specifically, computing device 11 compares the partial optimal positions of the members determined in steps S2 to S4 with an overall optimal position estimated through estimation model 30D to determine whether the partial optimal positions are an optimal position when the total balance of the entire dental implant 70 is considered. For example, computing device 11 may determine, based on a condition of whether a position allows each member to be assembled together as one dental implant, whether the partial optimal positions of the members are an optimal position when the members are viewed as a whole. A position allowing each member to be assembled together as one dental implant means a position allowing each member to be interconnected to function as dental implant 70. The position allowing each member to be assembled together as one dental implant can be uniquely determined depending on the type of the member.

When at least one of the partial optimal positions of the members determined in steps S2 to S4 is not an optimal position (NO in step S6), estimation models 30A to 30C request estimation model 30D to adjust the partial optimal positions of top structure 71, abutment 72 and fixture 73, and estimation models 30A to 30C again perform steps S2 to S4 to determine post-adjustment partial optimal positions. The adjustment is to move and rotate the members' partial optimal positions in three dimensions.

For example, computing device 11 inputs the three-dimensional data acquired in step S1 to estimation model 30D to determine an overall optimal position for dental implant 70. Estimation model 30D acquires partial optimal positions output at estimation models 30A to 30C. When estimation model 30D determines that the partial optimal positions are not optimal positions, estimation model 30D for example replies to estimation models 30A to 30C with post-adjustment positional information allowing the partial optimal positions to approach the positions that top structure 71, abutment 72 and fixture 73 assume in the overall optimal position. Thus, estimation model 30D refers to a positional relationship between the current partial optimal positions of the members to cause the partial optimal positions of top structure 71, abutment 72 and fixture 73 to approach positions allowing the members to be assembled as one dental implant.

For example, when there is a distance of 50.0 µm between the partial optimal position of top structure 71 and the position of top structure 71 in the overall optimal position, estimation model 30D generates post-adjustment positional information to cause the partial optimal position of top structure 71 to approach the position of top structure 71 in the overall optimal position by 5.0 µm. That is, estimation model 30D causes estimation model 30A to perform adjustment to cause the partial optimal position of top structure 71 to approach the position of top structure 71 in the overall optimal position by 1%. Estimation model 30D replies to cause the partial optimal position of each member to approach the overall optimal position in accordance with a rate predetermined for the member.

Estimation models 30A to 30C determine, based on a critical position, whether the members' respective partial optimal positions are movable and/or rotatable as replied by estimation model 30D. The critical position is a position predetermined for each of top structure 71, abutment 72 and fixture 73 and means a position in which each member cannot be attached based on a predetermined aspect when the member is viewed independently. The predetermined critical position is for example a position away from a partial optimal position by a predetermined percentage (e.g., 1%).

For example, top structure 71 can have a critical position determined in view of occlusion and aesthetics. When the critical position for top structure 71 is considered in view of occlusion, computing device 11 considers top structure 71 alone, and determines that top structure 71 exceeds the critical position when a relative distance between the tooth axis of top structure 71 and the tooth axis of the opposing tooth and a relative positional relationship of each in inclination exceed a critical range in view of aesthetics. That is, the critical position for top structure 71 is determined based on a relative positional relationship between top structure 71 and the opposing teeth. For example, when an area of top structure 71 that is exposed from gingiva is larger than a predetermined area, computing device 11 may determine that the partial optimal position of top structure 71 exceeds the critical position.

Furthermore, abutment 72 and fixture 73 can each have a critical position determined in view of pain and tolerance. Computing device 11 determines critical positions for abutment 72 and fixture 73 in view of pain based on a relative positional relationship between the positions of abutment 72 and fixture 73 and nerves. When abutment 72 and fixture 73 have their critical positions set in view of tolerance, computing device 11 determines that the partial optimal positions of abutment 72 and fixture 73 exceed the critical positions when in a plan view of a jawbone an extension of the tooth axis of abutment 72 and fixture 73 passes through the jawbone at a location smaller in thickness than a predetermined thickness.

Estimation models 30A to 30C, in response to the reply, each move and/or rotate the partial optimal position of the respective member, and when the estimation model determines that the moved and/or rotated partial optimal position exceeds the critical position, the estimation model reduces an amount of moving and/or rotating the partial optimal position. With reference to the above-described example of top structure 71, when estimation model 30A determines that a partial optimal position of top structure 71 moved by 50.0 µm based on post-adjustment positional information exceeds the critical position, the estimation model causes the partial optimal position of top structure 71 to approach a position of top structure 71 in the overall optimal position by 5.0 µm rather than 50.0 µm, for example. That is, in response to the reply received from estimation model 30D, estimation model 30A causes the partial optimal position of top structure 71 to approach the position of top structure 71 in the overall optimal position by 0.01%. Subsequently, estimation models 30A to 30C each again request estimation model 30D to reply for whether the partial optimal position that estimation model 30A, 30B, 30C estimates is an optimal position while the total balance of the entire dental implant 70 is considered, and estimation model 30D replies to this request while again considering the overall optimal position. Thus, steps S2 to S6 are repeated.

That is, when computing device 11 determines that the partial optimal positions of the members updated in steps S2 to S4 are not optimal when the members are viewed as a whole (NO in step S6), estimation model 30D causes steps S2 to S5 to be performed again. Thus, computing device 11 causes the partial optimal position of each member to approach the overall optimal position as close as possible. Repeating steps S2 to S4 can update each partial optimal position to a position close to the overall optimal position with each predetermined aspect as a starting point. When such a request and reply are repeated a predetermined number of times and despite that each partial optimal position is not a position allowing one implant to be assembled, computing device 11 adjusts each current partial optimal position to be a position allowing one implant to be assembled without considering the overall optimal position. Specifically, computing device 11 uses the partial optimal position of one of the members as a reference and updates the partial optimal positions of the other members so that the other members can be connected to the member having its partial optimal position serving as the reference. As a result, finally, computing device 11 can arrange each partial optimal position at a position allowing one implant to be assembled.

When computing device 11 determines that the partial optimal positions of the members updated in steps S2 to S4 are an optimal position with the members viewed as a whole (YES in step S6), the computing device generates support information (step S7) and outputs the support information (step S8). Note that estimation model 30D may not perform the process of causing each partial optimal position to approach to the overall optimal position and may instead use the partial optimal position of one of the members as a reference and reply to move and/or rotate the partial optimal positions of the other members so that the other members are connectable to the member serving as the reference. In that case, a member corresponding to a predetermined aspect having a priority higher than other priorities can be determined as the member serving as the reference. For example, when the user considers that aesthetics is important for top structure 71, estimation model 30D may move and/or rotate the partial optimal positions of abutment 72 and fixture 73 with reference to the position of top structure 71 estimated through estimation model 30A.

Thus, in the present embodiment, each member's independently output partial optimal position serves as a reference and an optimal position with each member viewed as a whole is thus approached. This can derive as support information partial optimal positions allowing one implant to be assembled while reflecting each member's independently output partial optimal position. The operator can understand an appropriate position for attachment of dental implant 70 simply by seeing the support information, and support apparatus 1 of the first embodiment allows a position to be determined in an oral cavity for each member of the dental implant and furthermore, allows a position to be adjusted for at least one member based on a positional relationship of each member, so that the operator can optimally perform a procedure using the dental implant regardless of the operator's experiences.

### <Second Embodiment>

In the first embodiment, an example has been described to output support information using partial optimal positions estimated through estimation models 30A to 30C. However, support apparatus 1 may output the support information without using estimation models 30A to 30C, and in the second embodiment, an example will be described to generate the support information through rule-based processing that does not use so-called artificial intelligence such as a neural network.

While support apparatus 1 in the second embodiment, as well as in the first embodiment, also outputs support information through the Fig. 9 flowchart, steps S2 to S6 are performed through rule-based processing rather than estimation model 30. The rule-based processing described in the second embodiment is described in support program 20. Hereinafter the rule-based processing described in the second embodiment will be described with reference to Fig. 9. Note that any configuration of support apparatus 1 in the second embodiment that overlaps that of support apparatus 1 in the first embodiment will not be described repeatedly.

Computing device 11 is configured to be capable of determining a partial optimal position for each member for each predetermined aspect based on the three-dimensional data acquired in step S1. The predetermined aspect may for example be aesthetics, occlusion, tolerance, pain suffered by a patient, etc. The partial optimal position for each member can be determined on a rule basis using support program 20.

Computing device 11 determines a partial optimal position for each member independently based on the three-dimensional data acquired in step S1 and a predetermined rule. For example, in view of aesthetics, computing device 11 determines a partial optimal position for top structure 71 with reference to a gingival line in combined data. More specifically, when focusing on top structure 71 alone with respect to three-dimensional data representing a state with a missing tooth, computing device 11 determines that a position allowing top structure 71 to be the most aesthetic is a partial optimal position for top structure 71 for aesthetics. Computing device 11 determines a partial optimal position for top structure 71 for aesthetics for example in accordance with the gingival line in the combined data, a position relative to an adjacent tooth, a geometrical balance, etc. That is, a partial optimal position is a position obtained when a predetermined member is independently considered for a predetermined aspect while the other members and aspects are not considered as well as the partial optimal position. Note that in the second embodiment a partial optimal position can be determined using an average value or a median value based on data obtained from a past statistical result of three-dimensional data of a patient. Furthermore, in the second embodiment, a partial optimal position may be manually input by a user, and the input data may be used to train estimation models 30A to 30C described in the first embodiment to estimate a partial optimal position.

Similarly, computing device 11 determines in view of occlusion a partial optimal position for top structure 71 for occlusion with reference to an opposing tooth's position and tooth axis in the three-dimensional data. For example, computing device 11 determines that a position of top structure 71 that allows the tooth axis of the opposing tooth to align with the tooth axis of top structure 71 is a partial optimal position for top structure 71 for occlusion. The partial optimal position for top structure 71 for occlusion is a position determined without considering aesthetics for top structure 71.

Furthermore, computing device 11 determines a partial optimal position for abutment 72 in view of tolerance. Computing device 11 determines a partial optimal position for abutment 72 for tolerance on a condition that abutment 72 does not come into contact with a surface of gingiva when top structure 71 and fixture 73 are connected together. Furthermore, in view of pain and tolerance, computing device 11 determines that a position of fixture 73 allowing an extension line of the tooth axis of fixture 73 to pass through a thickest portion of a jawbone is a partial optimal position for fixture 73 for pain and tolerance. Computing device 11 can determine these partial optimal positions based on the three-dimensional data by executing support program 20. The condition for determining a partial optimal position may be predetermined as described above, or may be settable by the user.

In the second embodiment, computing device 11 prioritizes these predetermined aspects. For example, computing device 11 prioritizes these aspects to give highest priority to pain followed by tolerance, occlusion, and aesthetics. Alternatively, computing device 11 may prioritizes these aspects to give highest priority to tolerance followed by pain, occlusion, and aesthetics.

Furthermore, the prioritization of the predetermined aspects set by computing device 11 may be settable by the user. For example, the user sets a numerical value representing a priority for each aspect so that a sum of the priorities of the aspects is "100". Specifically, the user may set "40" for pain, "30" for tolerance, "20" for occlusion, and "10" for aesthetics. Computing device 11 determines that an aspect is given a high priority when a large numerical value is set for that aspect, and the computing device determines that an aspect is given a low priority when a small numerical value is set for that aspect. While the present embodiment adopts pain, tolerance, occlusion, and aesthetics as predetermined aspects, another aspect may be adopted and settable by the user.

Furthermore, a numerical value representing a priority for each aspect may be automatically determined by computing device 11 based on input data corresponding to the aspect, for example. For example, the user inputs a state of a disease of a dentition to support apparatus 1 as information corresponding to pain. A state of a disease of a dentition is information relevant to pain that would be caused after dental implant 70 is attached, and the state includes presence and/or absence of an early contact, a gingival state, a state of alveolar bone, etc. Computing device 11 automatically determines a numerical value representing a priority for pain based on a state of a disease of a dentition that is input. Specifically, when a state of a disease of a dentition that is input does not represent a good state, computing device 11 increases a numerical value representing a priority for pain, whereas when a state of the disease of the dentition that is input represents a good state, computing device 11 decreases the numerical value representing the priority for pain. Furthermore, the user may input information representing a state of periodontal disease as information corresponding to pain. A state of periodontal disease may be determined for example through another estimation model that estimates the state of periodontal disease from an image showing an interior of an oral cavity.

The user inputs a held state of a member to support apparatus 1 as information corresponding to tolerance. A held state of a member is information representing a state in which each member is held after the member is attached, and the state can be directions of tooth axes of opposing and adjacent teeth, a state of an alveolar bone that is a state of a trabecular bone, bone density, etc. Computing device 11 automatically determines a numerical value representing a priority for tolerance based on a held state of a member that is input. When information is received representing that a member is not held in a good state, computing device 11 increases a numerical value representing a priority for tolerance, whereas when information is received representing that the member is held in a good state, computing device 11 decreases the numerical value representing the priority for tolerance.

The user inputs an occlusal state of an opposing tooth to support apparatus 1 as information corresponding to occlusion. An occlusal state of a tooth is information representing whether occlusion with an opposing tooth is easily achieved, and the state can include the opposing tooth's tooth axis, shape, etc. Based on an occlusal state of a tooth that is input, computing device 11 automatically determines a numerical value representing a priority for occlusion. Computing device 11 increases a numerical value representing a priority for occlusion when information is received representing that occlusion with the opposing tooth is not easily achieved, whereas the computing device decreases the numerical value representing the priority for occlusion when information is received representing that occlusion with the opposing tooth is easily achieved. Furthermore, the user may input jaw motion data and/or a result of an output of occlusion simulation software (not shown) as information corresponding to occlusion.

As information corresponding to aesthetics, the user inputs a positional relationship between a plurality of adjacent teeth, a positional relationship between a plurality of opposing teeth, or a gingival state to support apparatus 1. Computing device 11 automatically determines a numerical value representing a priority for aesthetics based on the positional relationship between the plurality of adjacent teeth, the positional relationship between the plurality of opposing teeth, or the gingival state, that is input. Computing device 11 increases a numerical value representing a priority for aesthetics when it is determined from the input data that it is of high necessity to place importance on aesthetics, whereas the computing device decreases the numerical value representing the priority for aesthetics when it is determined from the input data that it is of low necessity to place importance on aesthetics. Note that the user may input data representing a facial shape of the user that is scanned with a face scanning device (not shown) as information corresponding to aesthetics. In that case, computing device 11 may determine a priority for aesthetics with reference to the position of the cheeks of the user. Support apparatus 1 may use an estimation model to determine a priority for each aspect for determining the priority for the aspect.

According to the second embodiment, computing device 11 in step S2 determines a partial optimal position for top structure 71 in view of aesthetics and a partial optimal position for top structure 71 in view of occlusion. Furthermore, in step S3, computing device 11 determines a partial optimal position for abutment 72 in view of tolerance. Furthermore, in step S4, computing device 11 determines a partial optimal position for fixture 73 in view of pain and tolerance. In step S5, computing device 11 acquires a positional relationship between the partial optimal positions of the members for each aspect.

In step S6, computing device 11 determines whether each partial optimal position is an optimal position when the members are viewed as a whole. In the second embodiment, top structure 71 has partial optimal positions determined for two aspects, and accordingly, in step S6, when one of the partial optimal positions of top structure 71 can be connected to the partial optimal position of abutment 72 and that of fixture 73, computing device 11 can determine that each partial optimal position is an optimal position with the members viewed as a whole.

In the second embodiment, when it is determined that each partial optimal position does not allow the respective member to be attached to assemble one implant (NO in step S6), computing device 11 for example determines an overall optimal position that serves as a center for each partial optimal position based on a positional relationship of the partial optimal positions of the members for each aspect rather than using an overall optimal position obtained through estimation model 30D. While in the first embodiment, estimation model 30D is used to estimate an overall optimal position, in the second embodiment, computing device 11 determines a position output on a rule basis to serve as a center for each partial optimal position, and the computing device determines that a position allowing one implant to be assembled at the position serving as the center is an overall optimal position.

Subsequently, computing device 11 according to the second embodiment repeats steps S2 to S6, similarly as done in the first embodiment, to gradually adjust each current partial optimal position to approach the overall optimal position that serves as the center for each partial optimal position. As well as in the first embodiment, computing device 11 causes each partial optimal position to approach the overall optimal position that serves as the center for the partial optimal position at a predetermined rate.

In the second embodiment, computing device 11 may determine the predetermined rate of causing each partial optimal position to approach the overall optimal position in accordance with a degree of priority set for an aspect as described above. Computing device 11 may decrease an amount of movement and an amount of rotation for a partial optimal position corresponding to an aspect with a high priority set, and the computing device may increase an amount of movement and an amount of rotation for a partial optimal position corresponding to an aspect with a low priority set.

For example, when predetermined aspects are prioritized such that a highest priority is given to pain followed by tolerance, occlusion and aesthetics, computing device 11 moves and rotates a partial optimal position for fixture 73 in view of pain and tolerance so as to approach the overall optimal position in the second embodiment by 0.1%. Furthermore, computing device 11 moves and rotates a partial optimal position for abutment 72 in view of tolerance so as to approach the overall optimal position in the second embodiment by 0.2%. Furthermore, computing device 11 moves and rotates a partial optimal position for top structure 71 in view of occlusion so as to approach the overall optimal position in the second embodiment by 0.5%. Furthermore, computing device 11 moves and rotates a partial optimal position for top structure 71 in view of aesthetics so as to approach the overall optimal position in the second embodiment by 0.8%. Thus, computing device 11 according to the second embodiment updates each partial optimal position. Based on each updated partial optimal position, computing device 11 again calculates an overall optimal position that serves as a center for each partial optimal position to also update the overall optimal position that serves as the center for the partial optimal position. Thus, in the second embodiment, when each partial optimal position is caused to approach an overall optimal position, the partial optimal position is moved and rotated at a rate corresponding to a numerical value set as a priority set as a numerical value for a predetermined aspect. In the first embodiment as well, each partial optimal position may be moved and/or rotated according to a numerical value set as a priority for a predetermined aspect.

When steps S2 to S6 are repeated a predetermined number of times or more and despite that one implant cannot be assembled, then, as has been described in the first embodiment, computing device 11 sets one of the partial optimal positions as a reference partial optimal position and moves and/or rotates the other partial optimal positions so as to align with the reference partial optimal position. When each partial optimal position allows one implant to be assembled (YES in step S6), computing device 11 generates as support information a position for dental implant 70 based on the finally updated partial optimal position of each member (step S7) and outputs the support information (step S8). Accordingly, support apparatus 1 according to the second embodiment also allows an operator to understand an appropriate position for attachment of dental implant 70 simply by seeing the support information, and can thus appropriately support a dental implant procedure.

### [Modification]

In support apparatus 1 according to the first and second embodiments, estimation models 30A to 30D are models to estimate a position for each member. However, in addition to estimating a position for each member based on three-dimensional data representing a state with a missing tooth, estimation models 30A to 30D may be models capable of estimating a base material for the member, a model for the member, a manufacturer of the member, and a combination of the member as determined by the manufacturer. Support apparatus 1 can thus output not only a position for dental implant 70 but also an optimal type of each member of dental implant 70 as the support information.

For step S1, three-dimensional data in which at least one tooth is missing is input by way of example. However, the data input in step S1 may be three-dimensional data in which no tooth is missing. In that case, the user subjects teeth on a dental arch to segmentation to generate new three-dimensional data representing a state in which a specific tooth specified by the user is missing. Computing device 11 may estimate a position for each member while considering that dental implant 70 is to be attached in a location where the specific tooth is missing. Furthermore, computing device 11 may automatically detect a position at which dental implant 70 should be inserted, or the computing device may estimate a position for each member as the dental implant is to be inserted at a position designated by the user. For example, when three-dimensional data representing a state with a plurality of teeth missing is input, the user may input to support apparatus 1 information representing in which location support apparatus 1 is caused to estimate to position and insert dental implant 70.

As described above, in the first embodiment, an example has been described to estimate a partial optimal position and an overall optimal position through estimation models 30A to 30D, and in the second embodiment, an example has been described to determine a partial optimal position and an overall optimal position through support program 20 on a rule basis. Whether estimation model 30 is used or decision is made on a rule basis may be determined for each partial optimal position and each overall optimal position. For example, a partial optimal position for top structure 71 may alone be estimated through estimation model 30A, and partial optimal positions for abutment 72 and fixture 73 may be determined on a rule basis. Alternatively, each partial optimal position may be determined on a rule basis, and an overall optimal position may alone be estimated through estimation model 30D.

It should be understood that the presently disclosed embodiments are illustrative and non-restrictive in any respect. The scope of the present disclosure is defined by the terms of the claims rather than the above description, and is intended to encompass any modification falling within the meaning and scope equivalent to the terms of the claims. Note that the configurations represented by way of example in the present embodiments and the configurations represented by way of example in the modifications can be combined as appropriate.

## Claims

1. A support apparatus configured to support a procedure using a dental implant including a plurality of members, the support apparatus comprising:
an acquisition unit configured to acquire three-dimensional data representing a three-dimensional geometry of an interior of an oral cavity including teeth forming a dentition and a jawbone;
a computing unit configured to determine a position for each of the plurality of members in the oral cavity based on the three-dimensional data acquired by the acquisition unit, and adjust the determined position of at least one member of the plurality of members based on a positional relationship between the plurality of members; and
an output unit configured to output support information representing a position for each of the plurality of members after the adjustment performed by the computing unit.

2. The support apparatus according to claim 1, wherein the positional relationship follows a priority of a predetermined aspect.

3. The support apparatus according to claim 1 or 2, wherein the three-dimensional data includes CT data obtained through computer tomography of the dentition and the jawbone.

4. The support apparatus according to claim 3, wherein the three-dimensional data includes combined data composed of optical scanner data and the CT data combined together with reference to a position of a tooth crown, the optical scanner data including positional information of each point of point cloud data representing a surface of the dentition.

5. The support apparatus according to any one of claims 1 to 4, wherein the computing unit is configured to determine a position for a specific member of the plurality of members based on the three-dimensional data by using a first estimation model, the first estimation model being trained to determine a partial optimal position for the specific member based on the three-dimensional data.

6. The support apparatus according to any one of claims 1 to 5, wherein the computing unit is configured to adjust the position of the at least one member by using a second estimation model, the second estimation model being trained based on the three-dimensional data to estimate a position for each of the plurality of members such that an overall position for the plurality of members is an overall optimal position.

7. The support apparatus according to any one of claims 1 to 6, wherein the computing unit is configured to adjust the position of the at least one member based on a set value set by a user to determine a position for each of the plurality of members, or a predetermined set value.

8. The support apparatus according to claim 2, wherein
the predetermined aspect includes at least one of: pain of a subject to be treated; tolerance of the plurality of members; occlusion of the dentition; and aesthetics in the oral cavity, and
a numerical value representing a priority is set for each of the pain, the tolerance, the occlusion, and the aesthetics.

9. The support apparatus according to claim 8, wherein
the pain includes a state of a disease of the dentition,
the tolerance includes a held state of the plurality of members,
the occlusion includes an occlusal state of opposing teeth,
the aesthetics includes a positional relationship between a plurality of adjacent teeth, a positional relationship between the opposing teeth, or a gingival state, and
the numerical value set for each of the pain, the tolerance, the occlusion, and the aesthetics is changeable by a user according to a state of each of the pain, the tolerance, the occlusion, and the aesthetics, respectively, or the positional relationship.

10. The support apparatus according to claim 9, wherein the aesthetics further includes a facial shape of the user scanned with a face scanning device.

11. The support apparatus according to claim 8 or 9, wherein the predetermined aspect is prioritized such that a highest priority is given to the pain followed by the tolerance, the occlusion, and the aesthetics or to the pain followed by the occlusion, the tolerance, and the aesthetics.

12. The support apparatus according to any one of claims 8 to 11, wherein the predetermined aspect is settable by a user.

13. The support apparatus according to claim 5, wherein
the first estimation model is further trained to determine a type of the specific member based on the three-dimensional data, and
the computing unit is configured to determine the type of the specific member based on the three-dimensional data by using the first estimation model.

14. The support apparatus according to claim 13, wherein the type of the specific member includes at least one of: a manufacturer of the specific member; and a combination of the plurality of members as determined by the manufacturer.

15. The support apparatus according to any one of claims 1-14, wherein the plurality of members includes a top structure and a fixture.

16. The support apparatus according to claim 15, wherein the plurality of members further includes an abutment.

17. A method for supporting by a computer a procedure using a dental implant including a plurality of members, the computer performing the steps of:
acquiring three-dimensional data representing a three-dimensional geometry of an interior of an oral cavity including teeth forming a dentition and a jawbone;
determining a position for each of the plurality of members in the oral cavity based on the three-dimensional data acquired;
adjusting, based on a positional relationship between the plurality of members, the position of at least one member of the plurality of members determined in the step of determining; and
outputting support information representing a position for each of the plurality of members after the adjustment.

18. A support program configured to support by a computer a procedure using a dental implant including a plurality of members, the program causing the computer to perform the steps of:
acquiring three-dimensional data representing a three-dimensional geometry of an interior of an oral cavity including teeth forming a dentition and a jawbone;
determining a position for each of the plurality of members in the oral cavity based on the three-dimensional data acquired;
adjusting, based on a positional relationship between the plurality of members, the position of at least one member of the plurality of members determined in the step of determining; and
outputting support information representing a position for each of the plurality of members after the adjustment.
